Europäisches Patentamt

European Patent Office

Office européen des brevets

(11) Numéro de publication: **0 317 439**
**A1**

## (12) DEMANDE DE BREVET EUROPEEN

(21) Numéro de dépôt: **88402900.0**

(22) Date de dépôt: **18.11.88**

(51) Int. Cl.⁴: **G 01 N 33/96**

(30) Priorité: **20.11.87 FR 8716314**

(43) Date de publication de la demande:
**24.05.89 Bulletin 89/21**

(84) Etats contractants désignés:
**AT BE CH DE ES FR GB GR IT LI LU NL SE**

(71) Demandeur: **BIO-FRANCE REACTIFS**
**15, Rue du Bois de la Champelle Parc d'Activités de Brabois**
**F-54500 Vandoeuvre-les-Nancy (FR)**

(72) Inventeur: **Nabet, Pierre**
**7, Rue Catherine de Bourbon**
**F-54140 Jarville (FR)**

(74) Mandataire: **Ores, Irène et al**
**CABINET ORES 6, Avenue de Messine**
**F-75008 Paris (FR)**

(54) **Sérum de nature humaine obtenu à partir de fractions résultant du fractionnement de sang ou de plasma humain, son procédé de préparation et réactif de laboratoire contenant ledit sérum.**

㉟ Sérum de nature humaine obtenu à partir de fractions résultant du fractionnement de sang ou de plasma humain, un réactif de laboratoire contenant ledit sérum, ainsi que son procédé de préparation.

Ce sérum de nature humaine est reconstitué à partir d'au moins une sous-fraction résiduelle résultant du fractionnement du plasma humain et/ou du sang humain et/ou du sang placentaire ou rétroplacentaire et en ce qu'il présente des caractéristiques chimiques et physicochimiques semblables à celles d'un sérum humain naturel, notamment en ce qui concerne la limpidité, la couleur, la teneur en différents constituants et le profil électrophorétique.

EP 0 317 439 A1

## Description

## SERUM DE NATURE HUMAINE OBTENU A PARTIR DE FRACTIONS RESULTANT DU FRACTIONNEMENT DE SANG OU DE PLASMA HUMAIN, SON PROCEDE DE PREPARATION ET REACTIF DE LABORATOIRE CONTENANT LEDIT SERUM.

La présente invention est relative à un sérum de nature humaine obtenu à partir de fractions résultant du fractionnement de sang ou de plasma humain, à un réactif de laboratoire contenant ledit sérum, ainsi qu'à son procédé de préparation.

On sait que l'utilisation de standards d'origine humaine (sérums ou plasmas de contrôle) est obligatoire en biologie clinique chaque fois que des tests de détermination de paramètres, utilisent une méthode de nature immunologique, ou lorsqu'il s'agit de mettre en évidence une activité biologique spécifique de nature humaine, comme c'est le cas, notamment des enzymes, des facteurs de la coagulation ou des facteurs du complément.

On sait que la plupart des standards humains actuellement utilisés sont des sérums humains complets naturels ou du sang placentaire ou rétroplacentaire. Or, tous ces produits sont de disponibilité réduite, de coût élevé et ils sont de plus réservés à des usages thérapeutiques.

Des sérums étalons ont été proposés dans l'Art antérieur et proviennent essentiellement de sang complet. En particulier :

- la demande de brevet européen n° 0 131 826 décrit un sérum de contrôle à base de sérum humain ou animal, associé à un tampon qui permet d'obtenir un pH physiologique. L'utilisation d'un tel tampon donne une certaine stabilité ;

- le brevet américain n° 4 158 544 enseigne un procédé de préparation de sérum étalon par traitement d'un plasma citraté, qui est d'abord décitraté, puis coagulé, le caillot de fibrine étant éliminé après concentration et le sérum concentré étant additionné d'un alkylènepolyol.

Toutefois, les sérums proposés résultent d'un pool limité en ce qui concerne les teneurs de ces constituants, en sorte que la stabilité de la composition est aléatoire d'un lot à l'autre.

Dans les centres de fractionnement du plasma humain, c'est généralement la méthode de COHN - ou toute méthode qui en est dérivée - qui est utilisée pour obtenir les fractions utilisées en thérapeutique telles que facteurs de la coagulation, immunoglobulines, albumine, fibrinogène. Au cours de ces fractionnements, certaines fractions, sous forme de pâtes ou de surnageants liquides, ne sont pas utilisées en thérapeutique et sont généralement détruites par incinération.

Compte-tenu de la difficulté de disposer facilement d'un sérum humain entier pour une utilisation non thérapeutique, notamment comme réactifs, de la difficulté de disposer de grandes quantités de sang placentaire et du coût important de ces produits, les Inventeurs se sont donné pour but de pourvoir à un substitut d'un tel sérum, provenant du fractionnement du sang ou du plasma humain, qui répond aux besoins de la pratique tant au niveau du coût que des quantités disponibles, tout en présentant une grande stabilité tant dans sa composition que dans son aptitude à sa conservation.

En effet, le nombre de dosages immunologiques effectués par les laboratoires d'analyses médicales est en augmentation considérable du fait de l'évolution des méthodes de diagnostic. Il en résulte un accroissement de la demande en sérums de calibration ou de contrôle qui, pour ce type de dosages, doivent nécessairement être des produits d'origine humaine (immunoprécipitation, immunonéphélométrie, immunoenzymologie ou radioimmunologie).

La présente invention a pour objet un sérum de nature humaine, caractérisé en ce qu'il est reconstitué à partir d'au moins une sous-fraction résiduelle résultant du fractionnement du plasma humain et/ou du sang humain et/ou du sang placentaire ou rétroplacentaire et en ce qu'il présente des caractéristiques chimiques et physico-chimiques semblables à celles d'un sérum humain naturel, notamment en ce qui concerne la limpidité, la couleur, la teneur en différents constituants et le profil électrophorétique.

Il y a lieu de rappeler que le fractionnement de COHN, tel qu'illustré sur les schémas I et II annexés, comprend un certain nombre d'étapes qui seront brièvement décrites ci-après :

- lorsque du plasma additionné d'une solution d'ACD (acide citrique, citrate de sodium, sel de calcium, glucose et eau) est traité par de l'éthanol à 10 % à -2 C, il donne lieu à une "pâte I" à partir de laquelle on récupère du fibrinogène et à une sous-fraction dite "surnageant I" ;

- lorsque du plasma additionné d'ACD est cryoprécipité à -40 C, on obtient un cryoprécipité qui contient le facteur VIII et le facteur super VIII et un "surnageant du cryoprécipité" ;

- les surnageants de ces traitements, réunis et traités à l'éthanol à 19 %, à pH 5,85 et à -5°C donnent lieu :

. d'une part à une "pâte II" qui, par traitement par de l'éthanol à 13 %, à pH 5,10 et à -30°C, donne lieu à une "pâte III" qui est une sous-fraction résiduelle qui est non-utilisée et à un surnageant III qui par traitement par l'éthanol à 25 %, à pH 7,2 et à -7°C, en présence de NaCl 3 g/l, permet de récupérer les immunoglobulines ;

. d'autre part à un surnageant II, qui par traitement à l'éthanol à 38 %, à pH 6,10 et à -5°C, donne lieu à une "pâte IV" qui est une sous-fraction résiduelle qui est non-utilisée et à un surnageant IV qui par traitement par l'éthanol à 41 %, à pH 4,85 et à -12°C, donne lieu à une "pâte V", essentiellement constituée par de l'albumine et à un surnageant V, sous-fraction résiduelle qui est non-utilisée.

Or, les Inventeurs se sont aperçus que les sous-fractions dites pâte III, pâte IV et surnageant V présentent des propriétés précieuses, en ce qu'elles permettent seules ou en combinaisons entre elles de reconstituer un sérum de nature humaine dont les applications sont nombreuses.

En conséquence, selon un mode de réalisation avantageux du sérum reconstitué conforme à l'invention, celui-ci est caractérisé en ce qu'il est reconstitué à partir de la sous-fraction dite pâte III.

Selon un autre mode de réalisation avantageux du sérum reconstitué conforme à l'invention, celui-ci est caractérisé en ce qu'il est reconstitué à partir de la sous-fraction dite pâte IV.

Selon encore un autre mode de réalisation avantageux du sérum reconstitué conforme à la présente invention, celui-ci est reconstitué à partir d'un mélange des sous-fractions dites pâte III et pâte IV.

Selon un autre mode de réalisation avantageux du sérum reconstitué conforme à l'invention, celui-ci est reconstitué à partir de la sous-fraction dite pâte III et/ou de la sous-fraction dite pâte IV, associée(s) à un apport d'albumine.

Cet apport d'albumine peut avantageusement être constitué par la sous-fraction dite surnageant V.

Cet apport d'albumine peut également être une solution d'albumine humaine pyrogène non-utilisée en thérapeutique ou une solution d'albumine d'origine animale, notamment une solution d'albumine bovine.

Selon encore un autre mode de réalisation avantageux du sérum reconstitué conforme à l'invention, celui-ci est reconstitué à partir de la sous-fraction dite pâte III et/ou de la sous-fraction dite pâte IV, à laquelle ou auxquelles est ajouté un apport d'albumine et/ou la sous-fraction dite surnageant fibrinogène et/ou les sous-frac tions dites éluats I et II et/ou la sous-fraction dite surnageant des immunoglobulines.

Selon un autre mode de réalisation du sérum reconstitué conforme à l'invention, celui-ci est sous forme lyophilisée.

Selon encore un autre mode de réalisation du sérum reconstitué, celui-ci est sous forme liquide.

Selon une disposition avantageuse de ce mode de réalisation, ledit sérum liquide est associé à au moins un conservateur approprié.

Selon encore un autre mode de réalisation du sérum reconstitué conforme à l'invention, celui-ci est sous forme congelée.

Selon un mode de réalisation avantageux du sérum reconstitué, celui-ci est formé d'un mélange en proportions variables des sous-fractions dites "pâte III", "pâte IV" et "surnageant V" convenablement traitées au préalable et dont les teneurs en constituants protéiques et en divers paramètres sont ajustées en fonction des proportions dudit mélange.

Selon une disposition avantageuse de ce mode de réalisation, les fractions issues de "pâte III", "pâte IV" et "surnageant V" sont présentes dans le mélange dans des proportions respectives en poids de 0-30 % environ de pâte III, de 0-30 % environ de pâte IV, de 50-70 % environ de surnageant V.

Le sérum reconstitué, conforme à la présente invention, présente les avantages suivants :
- il utilise les fractions habituellement rejetées par les Centres de Transfusion, et permet ainsi l'obtention d'un sérum à coût très réduit ;
- il correspond à un milieu standardisé, stable et reproductible, dont la composition est proche ou identique de celle du sérum humain physiologique.

La présente invention a egalement pour objet un réactif de laboratoire, caractérisé en ce qu'il est constitué par un sérum reconstitué conforme à l'invention.

Selon l'une des applications de ce réactif, il est utilisé pour les contrôles de qualité de sérum.

Selon une autre des applications de ce réactif, il est utilisé pour l'étalonnage des appareillages, dans les laboratoires de biologie et d'analyses médicales.

Le réactif conforme à l'invention peut notamment être utilisé comme matrice protéique pour l'étalonnage d'une substance spécifique qui lui est ajoutée en quantités connues.

La présente invention a également pour objet un procédé de préparation d'un sérum reconstitué, caractérisé en ce qu'au moins une sous-fraction dite pâte III et/ou pâte IV est homogénéisée dans un soluté physiologique approprié, en ce que la suspension obtenue est dialysée puis en ce que la suspension dialysée ainsi obtenue est clarifiée et sa concentration protéique est ajustée, de manière à obtenir un sérum reconstitué conforme à l'invention, mis sous une forme appropriée à sa conservation.

Selon un mode de mise en oeuvre avantageux de ce procédé de préparation, chaque sous-fraction pâteuse est dialysée contre un soluté physiologique identique ou différent de celui utilisé pour l'homogénéisation, à travers une membrane à zone de coupure d'au moins 1 000 daltons.

L'homogénéisation et la dialyse permettent d'éliminer les solvants organiques, d'équilibrer en ions et substrats et de redissoudre le maximum de protéines.

Selon un autre mode de mise en oeuvre avantageux de ce procédé de préparation, ladite suspension dialysée est clarifiée par ultracentrifugation.

Selon encore un autre mode de mise en oeuvre de ce procédé de préparation, ladite suspension dialysée est clarifiée par filtration.

Conformément à l'invention, la clarification est effectuée directement ou après précipitation préalable des lipoprotéines.

La précipitation des lipoprotéines est réalisée par toute technique connue, notamment par la méthode de BURNSTEIN-SCHOLNICK et MORFIN.

Selon un autre mode de mise en oeuvre de ce procédé, l'adjustement de la concentration en protéines est réalisé par ultrafiltration sur membrane à zone de coupure de 10 000 daltons, de manière à ce que la concentration protéique soit comprise entre 50 et 70 g/l.

Selon un autre mode de mise en oeuvre avantageux du procédé de préparation du sérum reconstitué, on ajoute un apport d'albumine à au moins une sous-fraction pâteuse traitée comme décrit ci-dessus.

Selon une disposition avantageuse de ce mode de mise en oeuvre, l'apport d'albumine est avantageusement constitué par la sous-fraction dite surnageant V, ledit surnageant étant concentré jusqu'à une valeur appropriée et dialysé contre un soluté physiologique approprié.

Selon une modalité avantageuse de cette disposition, le surnageant V est concentré par ultrafiltration sur membrane à zone de coupure de 10 000 daltons, de manière à obtenir une teneur en protéines comprise entre 30 et 80 g/1.

Selon un autre mode de mise en oeuvre, les différentes suspensions et/ou solutions obtenues sont mélangées en proportions variables, avantageusement de 0 à 30 % d'une solution obtenue à partir de la pâte III, de 0 à 30 % d'une solution obtenue à partir de la pâte IV et de 50 à 70 % d'une solution d'albumine, pour obtenir un sérum reconstitué.

Selon un autre mode de mise en oeuvre avantageux du procédé de préparation du sérum reconstitué conforme à l'invention, on ajoute en outre d'autres fractions résiduelles du fractionnement de COHN et notamment au moins l'une des fractions choisie dans le groupe qui comprend le "surnageant fibrinogène", les "éluats I et II" et "le surnageant des immunoglobulines".

Conformément à l'invention, ledit sérum reconstitué est en outre mis sous une forme convenable à sa conservation par lyophilisation, filtration stérilisante ou congélation.

Outre les dispositions qui précèdent, l'invention comporte encore d'autres dispositions, qui ressortiront de la description qui va suivre, qui se réfère à des examens de mise en oeuvre du procédé conforme à l'invention.

Il doit être bien entendu toutefois, que ces exemples sont donnés uniquement à titre d'illustration de l'objet de l'invention, dont ils ne constituent en aucune manière une limitation.

Exemple 1 - Préparation d'un sérum reconstitué conforme à l'invention :

1) Fractionnement selon la méthode de COHN du plasma humain prélevé sur ACD.

On procède au fractionnement comme montré sur les schémas I et II, qui reproduisent les étapes du processus de fractionnement de COHN.

Dans ces schémas, les fractions encadrées sont des sous-fractions résiduelles qui sont non-utilisées en thérapeutique et les fractions soulignées sont des fractions actuellement utilisées en thérapeutique.

De nombreuses autres variantes existent et, en particulier, des variantes dans lesquelles le polyéthylène glycol est utilisé à la place ou en plus de l'alcool, dans certaines étapes.

2) Préparation des pâtes III et IV

La pâte III est obtenue après isolement du fibrinogène, des facteurs VIII et super VIII et du PPSB.

La pâte IV est obtenue après isolement du fibrinogène, des facteurs VIII et super VIII, du PPSB et des immunoglobulines.

Les pâtes III et IV sont homogénéisées dans deux volumes d'un soluté physiologique approprié tel que le soluté de RINGER normal ou modifié comme suit NaCl 585 g ; KCl 37 g ; urée 30 g ; créatinine 1 g ; glucose 100 g ; $NaH_2PO_4$, $H_2O$ 6,8 g ; sulfate d'ammonium ferreux, 6 $H_2O$ 2 g ; $NaHCO_3$ 340 g ; $ZnSO_4$, $2H_2O$ 1,5 g ; $CuSO_4$ 1,25 g ; $MgCl_2$, 6 $H_2O$ 20 g ; la suspension obtenue est dialysée au moins 24 heures contre un soluté physiologique, à l'aide d'une membrane à seuil de coupure d'au moins 1 000 daltons, à raison de I litre de suspension pour 50 litres de liquide de contre-dialyse, ce qui permet l'élimination de l'alcool, la remise en solution d'un maximum des protéines, et l'équilibrage en ions et substrats.

Les suspensions obtenues sont clarifiées par centrifugation ou par filtration, soit directement, soit après précipitation des lipoprotéines par toute technique connue et, en particulier, par la méthode de BURNSTEIN-SCHOLNICK et MORFIN (1970) ; la concentration protéique est ajustée à environ 50 g/l par ultrafiltration sur membrane à zone de coupure de 10 000 daltons.

3) Préparation de la solution d'agent d'apport protéique supplémentaire :

La fraction surnageant V est obtenue en 1) après precipitation de l'albumine.

Elle est concentrée par ultrafiltration sur une membrane à zone de coupure de 10 000 daltons jusqu'à l'obtention de teneurs protéiques de 30 à 80 g/l ; la solution ainsi obtenue est ensuite dialysée, dans les mêmes conditions que pour les fractions III et IV, contre un soluté physiologique pour rééquilibrer en électrolytes et en substrats et pour éliminer le restant de l'alcool.

4) Préparation du sérum proprement dit.

On mélange avantageusement 50 à 70 % d'une solution obtenue à partir de la fraction surnageant V, avec 0 à 30 % d'une solution obtenue à partir de la pâte III et 0 à 30 % d'une solution obtenue à partir de la pâte IV.

On obtient un sérum reconstitué dont les caractéristiques sont reportées dans le tableau I.

Le sérum reconstitué, filtré stérilement sur une membrane 0,20 µm et conservé en flacon stérile a une conservation d'au moins 2 ans, comme montré également dans le tableau I.

TABLEAU I

| | Mise en service | 3ème mois | 6ème mois | 15ème mois | 21ème mois |
|---|---|---|---|---|---|
| Protides | 68 | 70 | 69 | 69 | 69 |
| IgG g/l | 3.0 | 4.1 | 3.2 | 3.3 | 4.2 |
| IgA g/l | 2.0 | 2.1 | 2.05 | 2.2 | 2.2 |
| IgM g/l | 1.5 | 1.5 | 1.55 | 1.56 | 1.55 |
| Transferrine | | 2.7 | 2.5 | 2.9 | 2.6 |
| Haptoglobine | 1.0 | | 1.0 | 1.01 | |
| Orosomucoïde | | > 0.5 | > 5.5 | > 2.72 | > 3 |
| $\alpha_1$ Antitrypsine | 0.8 | | 1.2 | 0.95 | |
| $\alpha_2$ Macroglobuline | 2.7 | 2.8 | 2.2 | 2.15 | 2.3 |
| Céruléoplasmine | 0.2 | 0.2 | 0.18 | 0.23 | 0.23 |
| C3 | 0.5 | 0.48 | 0.55 | 0.59 | 0.46 |
| RBP | 44 | 55 | 35 | 47 | 48 |
| HCG | 57 | 56 | 57 | 52.9 | |
| LH | 13.5 | 13.7 | 21 | 12.4 | 13 |
| FSH | 23.8 | 24.2 | 20.5 | 25.8 | 25 |
| STH | 3.7 | 3.5 | 3.3 | 3.7 | 3.4 |
| Insuline | 11 | 10 | 10 | 5 | 7 |
| Cortisol | 16 | 17 | 19 | 18 | 14.6 |
| Prolactine | 5 | 5 | | 5 | 3 |
| Ag Carcino. Embr. | 60 | 54 | 29 | 36 | 30 |
| Peptide C | 2.97 | 3.20 | 2.58 | 2.6 | 2.5 |
| $\alpha F$ toprotéine | 3 | 4 | 3 | 3 | 3 |
| Turbidité | 0.250 | | 0.260 | | 0.240 |

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre ni de la portée de la présente invention.

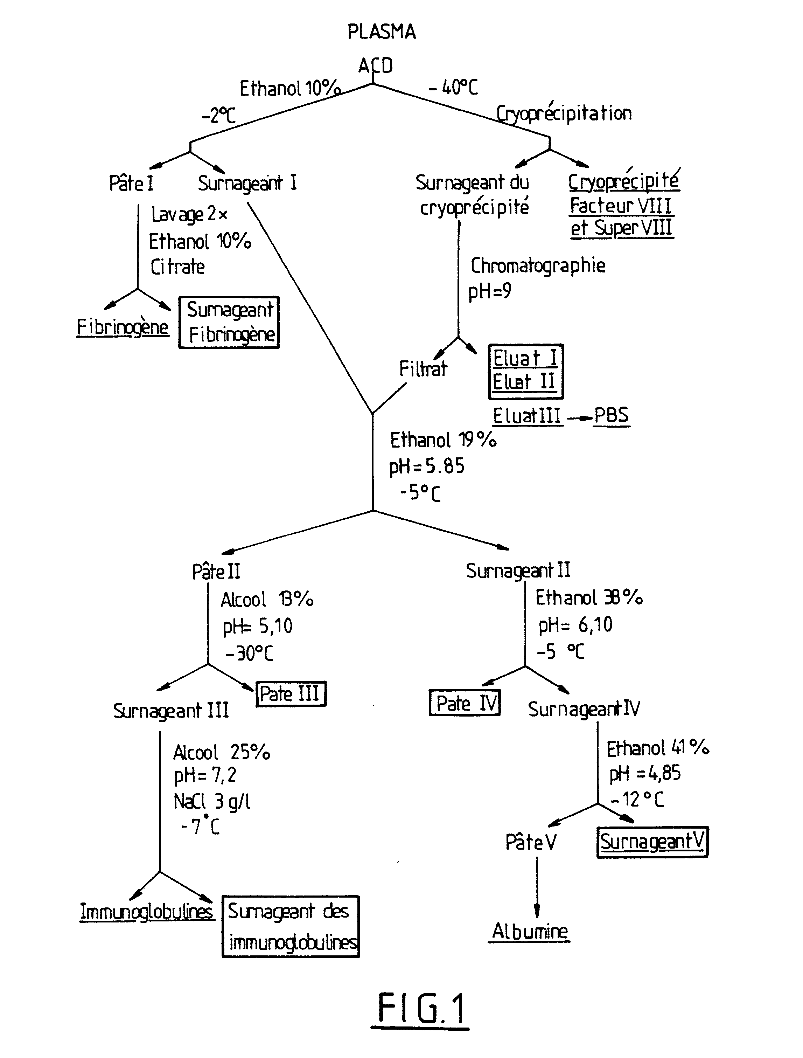

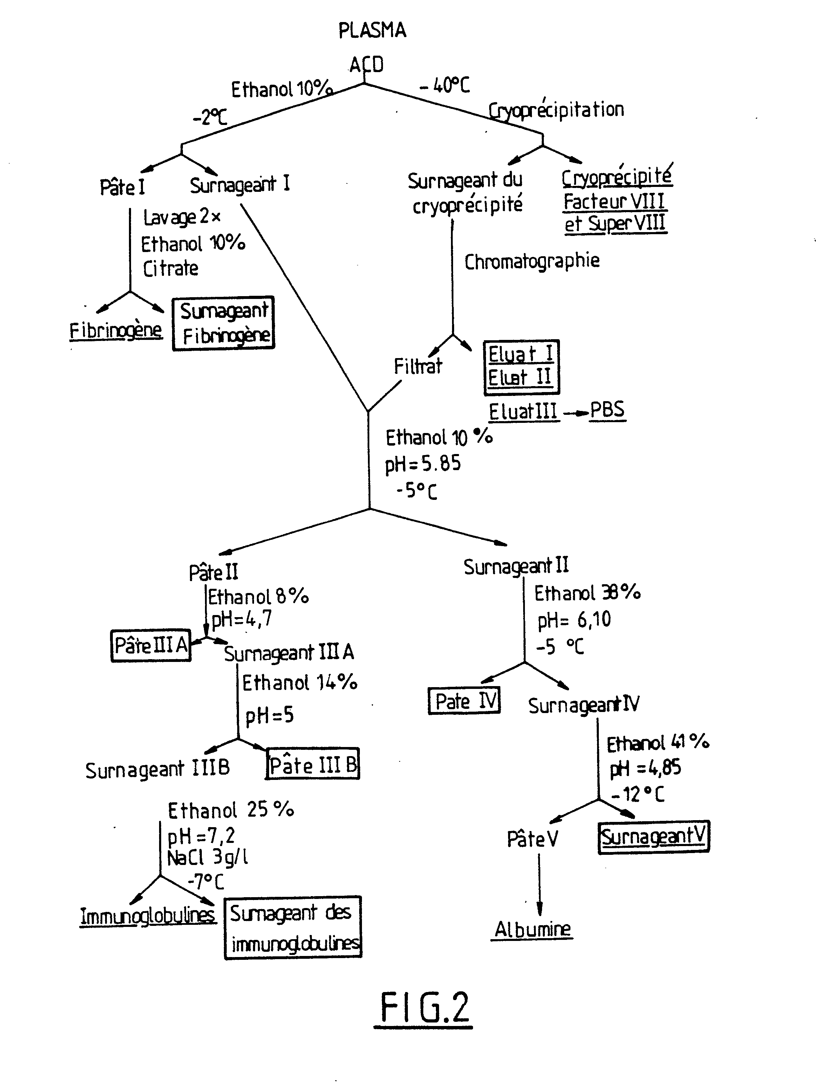

**Revendications**

1) Sérum de nature humaine, caractérisé en ce qu'il est reconstitué à partir d'au moins une sous-fraction résiduelle résultant du fractionnement du plasma humain et/ou du sang humain et/ou du sang placentaire ou rétroplacentaire et en ce qu'il présente des caractéristiques chimiques et physico-chimiques semblables à celles d'un sérum humain naturel, notamment en ce qui concerne la limpidité, la couleur, la teneur en différents constituants et le profil électrophorétique.

2°) Sérum reconstitué selon la revendication 1, caractérisé en ce qu'il est reconstitué à partir de la sous-fraction dite pâte III.

3°) Sérum reconstitué selon la revendication 1, caractérisé en ce qu'il est reconstitué à partir de la sous-fraction dite pâte IV.

4°) Sérum reconstitué selon la revendication 1, caractérisé en ce qu'il est reconstitué à partir d'un mélange des sous-fractions dites pâte III et pâte IV.

5°) Sérum reconstitué selon l'une quelconque des revendications 1 à 4, caractérisé en ce qu'il comprend en outre un apport d'albumine.

6°) Sérum reconstitué selon la revendication 5, caractérisé en ce que l'apport d'albumine est constitué par la sous-fraction dite surnageant V.

7°) Sérum reconstitué selon l'une quelconque des revendications 1 à 6, caractérisé en ce qu'il est reconstitué à partir de la sous-fraction dite pâte III et/ou de la sous-fraction dite pâte IV, à laquelle ou auxquelles est ajouté un apport d'albumine et/ou la sous-fraction dite surnageant fibrinogène et/ou la sous-fraction dite éluats I et II et/ou la sous-fraction dite surnageant des immunoglobulines.

8°) Sérum reconstitué selon l'une quelconque des revendications 1 à 7, caractérisé en ce qu'il est sous forme lyophilisée.

9°) Sérum reconstitué selon l'une quelconque des revendications 1 à 7, caractérisé en ce qu'il est sous forme liquide.

10°) Sérum reconstitué selon la revendication 9, caractérisé en ce que ledit sérum liquide est associé à au moins un conservateur approprié.

11°) Sérum reconstitué selon l'une quelconque des revendications 1 à 7, caractérisé en ce qu'il est sous forme congelée.

12°) Sérum reconstitué selon l'une quelconque des revendications 1 à 11, caractérisé en ce qu'il est formé d'un mélange en proportions variables des sous-fractions dites "pâte III", "pâte IV" et "surnageant V" convenablement traitées au préalable et dont les teneurs en constituants protéiques et en divers paramètres sont ajustées en fonction des proportions dudit mélange.

13°) Sérum reconstitué selon la revendication 12, caractérisé en ce que les fractions issues de "pâte III", "pâte IV" et "surnageant V" sont présentes dans le mélange dans des proportions respectives en poids de 0-30 % environ de pâte III, de 0-30 % environ de pâte IV, de 50-0 % environ de surnageant V.

14°) Réactif de laboratoire, caractérisé en ce qu'il est constitué par un sérum reconstitué selon l'une quelconque des revendications 1 à 13.

15°) Réactif selon la revendication 14, caractérisé en ce qu'il est appliqué aux contrôles de qualité.

16°) Réactif selon la revendication 14, caractérisé en ce qu'il est appliqué à l'étalonnage des appareillages, dans les laboratoires de biologie et d'analyses médicales.

17°) Procédé de préparation d'un sérum reconstitué selon l'une quelconque des revendications 1 à 13, ca ractérisé en ce qu'au moins une sous-fraction dite pâte III et/ou pâte IV est homogénéisée dans un soluté physiologique approprié, en ce que la suspension obtenue est dialysée puis en ce que la suspension dialysée ainsi obtenue est clarifiée et sa concentration protéique est ajustée, de manière à obtenir un sérum reconstitué selon l'une quelconque des revendications 1 à 13, mis sous une forme appropriée à sa conservation.

18) Procédé de préparation selon la revendication 17, caractérisé en ce que chaque sous-fraction pâteuse est dialysée contre un soluté physiologique identique ou différent de celui utilisé pour l'homogénéisation, à travers une membrane à zone de coupure d'au moins 1 000 daltons.

19°) Procédé de préparation selon l'une quelconque des revendications 17 et 18, caractérisé en ce que ladite suspension dialysée est clarifiée par ultracentrifugation.

20°) Procédé de préparation selon l'une quelconque des revendications 17 et 18, caractérisé en ce que ladite suspension dialysée est clarifiée par filtration.

21°) Procédé de préparation selon l'une quelconque des revendications 17 à 20, caractérisé en ce la clarification est effectuée directement sur la suspension dialysée.

22°) Procédé de préparation selon l'une quelconque des revendications 17 à 20, caractérisé en ce que la clarification est effectuée après précipitation préalable des lipoprotéines.

23°) Procédé de préparation selon l'une quelconque des revendications 17 à 22, caractérisé en ce que l'ajustement de la concentration en protéines est réalisé par ultrafiltration sur membrane à zone de coupure de 10 000 daltons, de manière à obtenir une concentration protéique comprise entre 50 et 70 g/l.

24°) Procédé de préparation selon l'une quelconque des revendications 17 à 23, caractérisé en ce qu'on ajoute un apport d'albumine à au moins une sous-fraction pâteuse traitée conformément au procédé selon l'une quelconque des revendications 17 à 23.

25°) Procédé de préparation selon la revendication 24, caractérisé en ce que l'apport d'albumine est constitué par la sous-fraction dite surnageant V, ledit surnageant étant concentré jusqu'à une valeur appropriée et dialysé contre un soluté physiologique approprié.

26°) Procédé de préparation selon l'une quelconque des revendications 24 et 25, caractérisé en ce que le surnageant V est concentré par ultrafiltration sur membrane à zone de coupure de 10 000 daltons, de manière à obtenir une teneur en protéines comprise entre 30 et 80 g/l.

27°) Procédé de préparation selon l'une quelconque des revendications 17 à 26, caractérisé en ce que les différentes suspensions et/ou solutions obtenues sont mélangées en proportions variables, avantageusement de 0 à 30 % d'une solution obtenue à partir de la pâte III, de 0 à 30 % d'une solution obtenue à partir de la pâte IV et de 50 à 70 % d'une solution d'albumine, pour obtenir un sérum reconstitué.

28°) Procédé de préparation selon l'une quelconque des revendications 17 à 27, caractérisé en ce qu'on ajoute en outre les autres fractions résiduelles du fractionnement de COHN et notamment au moins l'une des fractions choisie dans le groupe qui comprend la sous-fraction dite "surnageant fibrinogène", la sous-fraction dite "éluats I et II" et la sous-fraction dite " surnageant des immunoglobulines".

29°) Procédé de préparation selon l'une quelconque des revendications 17 à 28, caractérisé en ce que ledit sérum reconstitué est en outre mis sous une forme convenable à sa conservation par une méthode choisie dans le groupe qui comprend la lyophilisation, la filtration stérilisante et la congélation.

PLASMA

ACD

Ethanol 10%     -40°C

-2°C          Cryoprécipitation

Pâte I    Surnageant I        Surnageant du     Cryoprécipité
                              cryoprécipité      Facteur VIII

Lavage 2×                                 et Super VIII

Ethanol 10%

Citrate

                                Chromatographie

                                pH=9

Fibrinogène    | Surnageant                      | Eluat I |
              | Fibrinogène |    Filtrat          | Eluat II |

                                  Eluat III  →  PBS

Ethanol 19%

pH = 5.85

-5°C

Pâte II                          Surnageant II

Alcool 13%                        Ethanol 38%

pH= 5,10                          pH= 6,10

-30°C                             -5 °C

Surnageant III    | Pate III |      | Pate IV |    Surnageant IV

Alcool 25%                             Ethanol 41%

pH= 7,2                               pH =4,85

NaCl 3 g/l                             -12 °C

-7°C                          Pâte V     | Surnageant V |

Immunoglobulines    | Surnageant des
                      immunoglobulines |    Albumine

## FIG.1

EP 0 317 439 A1

PLASMA

ACD

Ethanol 10%  —40°C

-2°C  Cryoprécipitation

Pâte I  Surnageant I   Surnageant du   Cryoprécipité
                        cryoprécipité    Facteur VIII
                                         et Super VIII

Lavage 2×
Ethanol 10%
Citrate                 Chromatographie

Fibrinogène  | Surnageant |
             | Fibrinogène |

                              Filtrat   | Eluat I  |
                                        | Eluat II |

                                        Eluat III → PBS

                        Ethanol 10 %
                        pH = 5.85
                        -5°C

Pâte II                         Surnageant II
Ethanol 8%                      Ethanol 38%
pH = 4,7                        pH = 6,10
                                -5 °C
| Pâte III A |  Surnageant III A
              Ethanol 14%       | Pate IV |  Surnageant IV
              pH = 5
                                            Ethanol 41%
Surnageant III B  | Pâte III B |            pH = 4,85
                                            -12 °C
Ethanol 25 %
pH = 7,2
NaCl 3g/l                       Pâte V   | Surnageant V |
-7°C

Immunoglobulines | Surnageant des |      Albumine
                 | immunoglobulines |

## FIG.2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 158 544  (A.L. LOUDERBACK) --- | | G 01 N  33/96 |
| A | EP-A-0 131 826  (BOEHRINGER MANNHEIM) ----- | | |

**DOMAINES TECHNIQUES RECHERCHES (Int. Cl.4)**

G 01 N

Le présent rapport a été établi pour toutes les revendications

| Lieu de la recherche | Date d'achèvement de la recherche | Examinateur |
|---|---|---|
| LA HAYE | 19-12-1988 | MEYLAERTS H. |